(19) Europäisches Patentamt
European Patent Office
Office européen des brevets

(11) Numéro de publication : **0 421 831 B1**

(12) # FASCICULE DE BREVET EUROPEEN

(45) Date de publication du fascicule du brevet : **16.06.93 Bulletin 93/24**

(51) Int. Cl.$^5$ : **C07C 319/12,** C07C 323/52

(21) Numéro de dépôt : **90402408.0**

(22) Date de dépôt : **31.08.90**

(54) **Synthèse continue d'esters d'acides mercaptocarboxyliques.**

(30) Priorité : **06.10.89 FR 8913102**

(43) Date de publication de la demande : **10.04.91 Bulletin 91/15**

(45) Mention de la délivrance du brevet : **16.06.93 Bulletin 93/24**

(84) Etats contractants désignés : **AT BE CH DE DK ES FR GB GR IT LI LU NL SE**

(56) Documents cités : **DE-A- 1 693 111**

(73) Titulaire : **SOCIETE NATIONALE ELF AQUITAINE (PRODUCTION)**
**Tour Elf, 2, Place de la Coupole, La Défense 6**
**F-92400 Courbevoie (FR)**

(72) Inventeur : **Labat, Yves**
**35 Bis Rue Michel Houreau**
**F-64000 Pau (FR)**
Inventeur : **Muller, Jean-Pierre**
**3 Rue Casteret**
**F-64000 Pau (FR)**
Inventeur : **Litvine, Daniel**
**5 Rue du Pic du Midi d'Ossau**
**F-64230 Lescar (FR)**

(74) Mandataire : **Leboulenger, Jean et al**
**ATOCHEM Département Propriété Industrielle**
**Cédex 42- La Défense 10**
**F-92091 Paris la Défense (FR)**

EP 0 421 831 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne la fabrication d'esters d'acides mercaptocarboxyliques et, plus particulièrement, celle des esters octyliques de l'acide thioglycolique.

Le développement important de ces esters pour la préparation de stabilisants à l'étain des polymères chlorovinyliques rend hautement souhaitable leur production en continu et l'amélioration du rendement de la synthèse. D'autre part, la qualité des stabilisants dépend largement de celle des esters, notamment de leur pureté et de leur stabilité au stockage.

S'il paraît aisé d'estérifier un alcool formant un azéotrope avec l'eau tel que le 2-éthyl-hexanol ou des mélanges isomères d'alcools en $C_8$ (isooctanol), au moyen d'un acide organique thermiquement stable, il n'en est plus de même dans le cas d'un acide mercaptocarboxylique thermiquement instable tel que l'acide thioglycolique. En effet, par déshydratation, cet acide forme un oligomère estérifiable conduisant à la production de sous-produits indésirables selon le schéma :

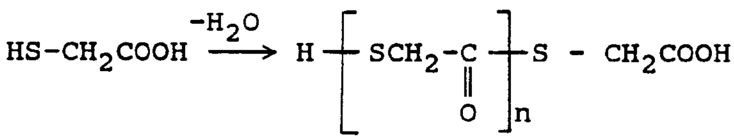


où n est majoritairement égal à 1 (dimère), mais peut aussi être supérieur à 1. On sait également que ce dimère linéaire peut aisément s'estérifier selon le schéma :

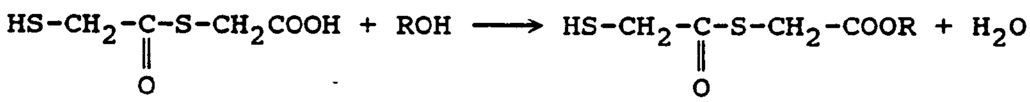

Ainsi, l'estérification d'un acide mercaptocarboxylique comme l'acide thioglycolique implique plusieurs réactions compétitives qui, selon les conditions opératoires retenues, peuvent provoquer la formation plus ou moins importante de sous-produits indésirables. En discontinu, on ne parvient pas à éviter la formation de dimères estérifiés et à obtenir en même temps une conversion élevée de l'acide mercaptocarboxylique.

Il a maintenant été trouvé qu'on peut grandement améliorer la sélectivité en l'ester désiré si l'on effectue l'estérification de manière continue en éliminant régulièrement l'eau formée de telle sorte que la concentration d'eau résiduelle dans le milieu réactionnel soit inférieure à 0,5 % en poids. On a en outre constaté de manière surprenante que, dans ces conditions, la durée de réaction est nettement inférieure à celle du procédé discontinu.

L'invention a donc pour objet un procédé de fabrication d'esters d'acides mercaptocarboxyliques et d'alcools formant un azéotrope avec l'eau, caractérisé en ce que l'estérification est réalisée en continu en présence d'un excès d'alcool(s) et que l'eau formée est éliminée, au fur et à mesure de sa formation, par entraînement azéotropique sous vide à une température telle que la concentration en eau résiduelle dans le milieu réactionnel reste constamment inférieure à 0,5 % en poids, de préférence égale ou inférieure à 0,1 % et avantageusement inférieure à 0,05 %.

Le procédé selon l'invention peut être mis en oeuvre dans un seul réacteur. Cependant, on préfère opérer dans deux réacteurs successifs (en série), le premier fonctionnant à une concentration en eau résiduelle comprise entre 0,2 et 0,5 % et le second à une concentration en eau résiduelle inférieure à 0,1 %, de préférence inférieure à 0,05 %.

L'alimentation en alcool et en acide mercaptocarboxylique du réacteur unique ou du premier réacteur dans le cas où l'on utilise deux réacteurs en série, doit être réglée de telle sorte que le rapport molaire alcool/acide mercaptocarboxylique soit compris entre 1,1 et 1,4. L'alcool entraîné avec l'eau est séparé de l'eau par décantation et recyclé au réacteur.

Comme catalyseur d'estérification on préfère utiliser l'acide sulfurique, mais on peut également employer d'autres catalyseurs d'estérification connus tels que, par exemple, l'acide méthanesulfonique, l'acide benzènesulfonique, l'acide p-toluènesulfonique. La quantité de catalyseur à mettre en oeuvre peut varier dans de larges limites sans influencer la sélectivité ; elle est généralement comprise entre environ 0,25 et 12,5 millimoles par mole d'acide mercaptocarboxylique et, de préférence, entre 1,25 et 5 millimoles.

La pression dans le ou les réacteurs est réglée de manière à obtenir une élimination satisfaisante de l'eau formée. Elle dépend non seulement de la valeur choisie pour la concentration en eau résiduelle, mais aussi de la température du milieu réactionnel, une température plus basse nécessitant une plus basse pression.

La température à laquelle est effectuée l'estérification selon l'invention peut varier dans de larges limites en fonction de la concentration en eau résiduelle choisie, de la pression appliquée et de la nature de l'alcool utilisé. En général, la température est avantageusement comprise entre environ 80 et 140°C. Dans le cas du 2-éthylhexanol et de l'isooctanol, on opère de préférence à une température de l'ordre de 120-130°C.

La concentration en eau résiduelle du milieu réactionnel peut être controlée de façon connue, par exemple par dosage infra-rouge.

Le temps de séjour des réactifs dans le(s) réacteur(s) peut varier dans de larges limites. Il est avantageusement compris entre 10 et 150 minutes, de préférence entre environ 30 et 80 minutes.

Bien que le procédé selon l'invention vise plus particulièrement la synthèse des esters octyliques de l'acide thioglycolique, il peut s'appliquer aussi à la fabrication d'esters dérivés d'autres acides mercaptocarboxyliques tels que, par exemple, l'acide thiolactique, l'acide 3-mercaptopropionique, et/ou dérivés d'autres alcools aliphatiques ou cycloaliphatiques en $C_2$ à $C_{18}$, de préférence les alcools en $C_4$ à $C_{12}$ comme, par exemple, le butanol, l'hexanol, le cyclohexanol, le décanol et le dodécanol.

Les exemples suivants illustrent l'invention sans la limiter. Les abréviations employées ont les significations suivantes :

**ATG** : acide thioglycolique :
$HS-CH_2-COOH$

**EHTG** : thioglycolate de 2-éthylhexyle :
$HS-CH_2-COO-CH_2CH(C_2H_5)-(CH_2)_3-CH_3$

**DIM** : dimère de l'ATG :
$HS-CH_2-CO-S-CH_2-COOH$

**DME** : ester 2-éthylhexylique du dimère de l'ATG :
$HS-CH_2-CO-S-CH_2-COO-CH_2CH(C_2H_5)-(CH_2)_3-CH_3$

**DSE** : dithiodiglycolate de 2-éthylhexyle :
$C_8H_{17}-OCO-CH_2-S-S-CH_2-COO-C_8H_{17}$

## EXEMPLE 1

On utilise un réacteur agité en verre d'un volume de 1,5 litre ayant un rapport hauteur/diamètre égal à 3,5, thermostaté au moyen d'une double enveloppe et muni des moyens connus de régulation de vide et de niveau. Il est relié à une pompe à vide par l'intermédiaire d'un condenseur pour séparer l'azéotrope eau-alcool, l'alcool étant recyclé au réacteur.

Ce réacteur est alimenté en continu avec 1250 g/heure d'un mélange contenant en poids 35 % d'ATG, 0,1 % de DIM, 64,8 % de 2-éthylhexanol et 0,1 % de $H_2SO_4$.

Après une phase de mise en régime permettant d'obtenir l'équilibre des paramètres de fonctionnement aux valeurs suivantes :

- temps de séjour : 55 minutes
- température : 130°C
- pression : 25,33 kPa (190 torr)
- indice d'acide (IA) : 8,6 mgKOH/g

on recueille dans le condenseur 81,4 g/heure d'une phase aqueuse contenant 0,44 % d'ATG et on soutire en continu 1160 g/heure d'un mélange dont l'analyse par acidimétrie (ATG), par RMN (ensemble des constituants organiques) et par IR (eau) permet de déterminer la composition suivante :

| | % en poids |
|---|---|
| ATG | 1,4 |
| Ethylhexanol | 17,45 |
| EHTG | 80,6 |

| | |
|---|---|
| DME | 0,2 |
| DSE | 0,1 |
| Eau | 0,25 |

ce qui correspond à un taux de conversion de l'ATG de 96 % et à une sélectivité en EHTG d'environ 99,4 %.

**EXEMPLES 2 A 5**

On opère comme à l'exemple mais en modifiant au moins un des paramètres de fonctionnement (temps de séjour, température, pression).

Les conditions opératoires et les résultats ainsi obtenus sont rassemblés, avec ceux de l'exemple 1, dans le tableau suivant.

| EXEMPLE | 1 | 2 | 3 | 4 | 5 |
|---|---|---|---|---|---|
| **CONDITIONS OPERATOIRES** | | | | | |
| - temps de séjour (mn) | 55 | 140 | 80 | 55 | 60 |
| - température (°C) | 130 | 130 | 130 | 120 | 130 |
| - pression (kPa) | 25,33 | 50,66 | 22 | 13,33 | 26,66 |
| - IA (mgKOH/g) | 8,6 | 9,4 | 5,8 | 8 | 7,5 |
| - Eau résiduelle (%) | 0,25 | 0,3 | 0,2 | 0,25 | 0,23 |
| **RESULTATS** | | | | | |
| Composition molaire du soutirat (%) : | | | | | |
| - EHTG | 74,4 | 72 | 74,2 | 75,1 | 78,0 |
| - DME | 0,15 | 0,1 | 0,16 | 0,2 | 0,2 |
| - Ethylhexanol | 25 | 25,3 | 24,9 | 24,1 | 21 |
| Conversion de l'ATG(%) | 96 | 95,6 | 97 | 96 | 96 |
| Sélectivité en EHTG(%) | 99,4 | 99,4 | 99,4 | 99,4 | 99,3 |

**EXEMPLE COMPARATIF 6** (essais en discontinu)

Dans un réacteur de 2 litres agité et thermostaté, on introduit 1600 g d'un mélange contenant environ en poids 39,4 % d'ATG, 0,1 % de DIM, 60,4 % de 2-éthylhexanol et 0,1 % de $H_2SO_4$. On chauffe ensuite à 110-130°C sous vide, la pression initiale de 100 kPa descendant jusqu'à 2,66 kPa après 7 heures.

Au bout de ce temps, le mélange réactionnel a un indice d'acide de 3,7 MgKOH/g et son analyse indique la composition molaire suivante :

| | |
|---|---|
| EHTG | 88% |
| DME | 1,09 % |
| Ethylhexanol | 10 % |

ce qui correspond à une conversion de l'ATG de 98 % et à une sélectivité en EHTG de 97,5 %.

Un second essai effectué dans les mêmes conditions, mais avec un rapport molaire éthylhexanol/ATG

plus élevé (1,2 au lieu de 1,1), c'est-à-dire en engageant 1600 g d'un mélange contenant en poids 37 % d'ATG, 0,1 % de DIM, 62,8 % de 2-éthylhexanol et 0,1 % de $H_2SO_4$, conduit après 8 heures de réaction aux résultats suivants :

| | |
|---|---|
| IA | 1,6 mgKOH/g |
| EHTG | 77% |
| DME | 0,7 % |
| Ethylhexanol | 22,1 % |

ce qui correspond à une conversion de l'ATG de 99 % et à une sélectivité en EHTG de 98,2 %.

Par comparaison avec les exemples 1 à 5 réalisés en continu, on constate que le mode discontinu est beaucoup plus long et conduit à la formation de DME.

## EXEMPLE 7

On opère dans deux réacteurs identiques à celui décrit à l'exemple 1 et disposés en série.

Le premier réacteur fonctionne exactement dans les mêmes conditions qu'à l'exemple 1. Le second réacteur est alimenté par le soutirat issu du premier réacteur (1160 g/h) et fonctionne dans les conditions suivantes :

| | |
|---|---|
| Temps de séjour | : 40 minutes |
| Température | : 130°C |
| Pression | : 9,33 kPa |
| Indice d'acide | : 1,2 mgKOH/g |

L'équilibre de marche étant atteint, on soutire en continu du second réacteur 1145 g/heure d'un produit ayant la composition suivante:

| | % en poids |
|---|---|
| ATG | 0,2 |
| Ethylhexanol | 15,1 |
| EHTG | 84,3 |
| DME | 0,27 |
| DSE | 0,08 |
| Eau | 0,04 |

ce qui correspond à un taux de conversion de l'ATG de 99,4 % et à une sélectivité en EHTG de 99,35 %.

## EXEMPLE 8

On opère comme à l'exemple 7 dans deux réacteurs en série et en remplaçant le 2-éthylhexanol par de l'isooctanol (mélange technique d'alcools isomères en $C_8$).

Le tableau suivant rassemble les conditions de fonctionnement et les résultats obtenus.

| | Premier réacteur | Second réacteur |
|---|---|---|
| **CONDITIONS OPERATOIRES** | | |
| - temps de séjour (minutes) | 55 | 50 |
| - température (°C) | 130 | 130 |
| - pression (kPa) | 26,66 | 13,33 |
| - IA (mgKOH/g) | 9,4 | 1,8 |
| - eau résiduelle (%) | 0,34 | 0,06 |
| **RESULTATS** | | |
| Composition molaire du soutirat (%) :<br>- thioglycolate d'isooctyle | 75,4 | 75,6 |
| - ester isooctylique du DIM | 0,15 | 0,2 |
| - isooctanol | 24,5 | 24 |
| Conversion de l'ATG (%) | 95 | 99 |
| Sélectivité en thioglycolate d'isooctyle (%) | 99,6 | 99,5 |

## EXEMPLE 9

On opère en continu comme à l'exemple 7 avec deux réacteurs en cascade.

On alimente le premier réacteur avec 1160 g/heure d'un mélange contenant en poids 47,6 % d'ATG, 0,4 % de DIM, 51,9 % de butanol et 0,1 % de $H_2SO_4$, et on opère dans les conditions suivantes :

Temps de séjour : 60 minutes
Température : 73°C
Pression : 10 kPa

En régime stabilisé, on élimine 80 g/heure de condensat aqueux et le soutirat obtenu alimente le second réacteur qui fonctionne dans les conditions suivantes :

Temps de séjour : 60 minutes
Température : 70°C
Pression : 6 kPa

A l'équilibre de marche, on récupère en continu du second réacteur un soutirat ayant la composition pondérale suivante :

| | |
|---|---|
| ATG | 0,6 % |
| Butanol | 15,5 % |
| Thioglycolate de butyle | 83,7 % |
| Eau | 0,1 % |

ce qui correspond à un taux de conversion de l'ATG de 98,5 % et à une sélectivité en thioglycolate de butyle supérieure à 99 %.

## EXEMPLE 10

On opère comme à l'exemple 7 avec deux réacteurs en série fonctionnant en continu dans les conditions

suivantes :

| | Premier réacteur | Second réacteur |
|---|---|---|
| Temps de séjour (minutes) | 60 | 60 |
| Température | 130°C | 140°C |
| Pression (kPa) | 26 | 10,6 |

Le premier réacteur est alimenté avec 2200 g/heure d'un mélange contenant en poids 25,1 % d'ATG, 0,2 % de DIM, 74,6 % d'isotridécanol et 0,1 % de $H_2SO_4$.

En régime stabilisé, on récupère en sortie du second réacteur un soutirat dont l'indice d'acide est de 1,6 mgKOH/g. La conversion de l'ATG est de 99 % et la sélectivité en thioglycolate d'isotridécyle est supérieure à 99 %.

## Revendications

1. Procédé de fabrication d'esters d'acides mercaptocarboxyliques et d'alcools formant un azéotrope avec l'eau, caractérisé en ce que l'estérification est réalisée en continu en présence d'un excès d'alcool(s) et que l'eau formée est éliminée, au fur et à mesure de sa formation, par entraînement azéotropique sous vide à une température telle que la concentration en eau résiduelle dans le milieu réactionnel reste constamment inférieure à 0,5 % en poids.

2. Procédé selon la revendication 1, dans lequel la concentration en eau résiduelle est égale ou inférieure à 0,1 % en poids et, de préférence, inférieure à 0,05 %.

3. Procédé selon la revendication 1, dans lequel on effectue l'estérification dans deux réacteurs successifs, le premier fonctionnant à une concentration en eau résiduelle comprise entre 0,2 et 0,5 % et le second à une concentration en eau résiduelle inférieure à 0,1 %.

4. Procédé selon l'une des revendications 1 à 3, dans lequel on opère à une température comprise entre environ 80 et 140°C.

5. Procédé selon l'une des revendications 1 à 4, dans lequel l'alimentation du réacteur unique ou du premier réacteur en alcool et en acide mercaptocarboxylique est réglée de telle sorte que le rapport molaire alcool/acide soit compris entre 1,1 et 1,4.

6. Procédé selon l'une des revendications 1 à 5, dans lequel le catalyseur d'estérification est l'acide sulfurique, l'acide méthanesulfonique, l'acide benzènesulfonique ou l'acide p-toluénesulfonique.

7. Procédé selon l'une des revendications 1 à 6, dans lequel l'acide mercaptocarboxylique est l'acide thioglycolique, l'acide thiolactique ou l'acide 3-mercaptopropionique.

8. Procédé selon l'une des revendications 1 à 7, dans lequel on utilise un alcool aliphatique ou cycloaliphatique en $C_2$ à $C_{18}$.

9. Procédé selon l'une des revendications 1 à 7, dans lequel on utilise le 2-éthylhexanol ou l'isooctanol et opère à une température de l'ordre de 120-130°C.

## Claims

1. Process for the production of esters of mercaptocarboxylic acids and alcohols forming an azeotrope with water, characterised in that the esterification is carried out continuously in the presence of an excess of alcohol(s) and in that the water formed is removed at the rate at which it is formed, by entraining azeotropically under vacuum at a temperature such that the residual water concentration in the reaction mixture remains constantly below 0.5% by weight.

2. Process according to Claim 1, in which the residual water concentration is equal to or below 0.1% by weight and preferably below 0.05%.

3. Process according to Claim 1, in which the esterification is carried out in two successive reactors, the first operating at a residual water concentration of between 0.2 and 0.5% and the second at a residual water concentration below 0.1%.

4. Process according to one of Claims 1 to 3, in which the reaction is carried out at a temperature between about 80 and 140°C.

5. Process according to one of Claims 1 to 4, in which the feed of alcohol and mercaptocarboxylic acid to the single reactor or to the first reactor is controlled in such a way that the molar ratio of alcohol/acid is between 1.1 and 1.4.

6. Process according to one of Claims 1 to 5, in which the esterification catalyst is sulphuric acid, methanesulphonic acid, benzenesulphonic acid or p-toluenesulphonic acid.

7. Process according to one of Claims 1 to 6, in which the mercaptocarboxylic acid is thioglycolic acid, thiolactic acid or 3-mercaptopropionic acid.

8. Process according to one of Claims 1 to 7, in which a $C_2$ to $C_{18}$ aliphatic or cycloaliphatic alcohol is used.

9. Process according to one of Claims 1 to 7, in which 2-ethylhexanol or isooctanol is used and the reaction is carried out at a temperature of the order of 120-130°C.

**Patentansprüche**

1. Verfahren zur Herstellung von Estern aus Mercaptocarbonsäuren und aus Alkoholen, die ein Azeotrop mit Wasser bilden, dadurch gekennzeichnet, daß die Veresterung kontinuierlich in Gegenwart eines Überschusses von Alkohol(en) durchgeführt wird, und das gebildete Wasser, in dem Maße wie es gebildet wird, durch Azeotrop-Destillation im Vakuum bei einer Temperatur eliminiert wird, so daß die Restwasserkonzentration im Reaktionsgemisch konstant unterhalb von 0,5 Gewichtsprozenten bleibt.

2. Verfahren nach Anspruch 1, bei dem die Konzentration an Restwasser gleich oder kleiner 0,1 Gewichtsprozente und bevorzugt kleiner als 0,05% ist.

3. Verfahren nach Anspruch 1, bei dem die Veresterung in zwei aufeinanderfolgenden Reaktoren durchgeführt wird, der erste bei einer Restwasserkonzentration zwischen 0,2 und 0,5%, und der zweite bei einer Restwasserkonzentration unterhalb von 0,1% arbeitend.

4. Verfahren nach einem der Ansprüche 1 bis 3, bei dem man bei einer Temperatur, die zwischen ungefähr 80 und 140°C liegt, arbeitet.

5. Verfahren nach einem der Ansprüche 1 bis 4, bei dem die Zugabe des Alkohols und der Mercaptocarbonsäure in den Einzelreaktor oder in den ersten Reaktor so eingeregelt ist, daß das molare Alkohol/Säure-Verhältnis zwischen 1,1 und 1,4 liegt.

6. Verfahren nach einem der Ansprüche 1 bis 5, bei dem der Veresterungskatalysator aus Schwefelsäure, Methansulfonsäure, Benzolsulfonsäure oder p-Toluolsulfonsäure besteht.

7. Verfahren nach einem der Ansprüche 1 bis 6, bei dem die Mercaptocarbonsäure Thioglykolsäure, Thiomilchsäure oder 3-Mercaptopropionsäure ist.

8. Verfahren nach einem der Ansprüche 1 bis 7, bei dem ein aliphatischer oder cycloaliphatischer $C_2$- bis $C_{18}$-Alkohol verwendet wird.

9. Verfahren nach einem der Ansprüche 1 bis 7, bei dem man 2-Ethylhexanol oder Isooctanol verwendet und bei einer Temperatur um 120-130°C arbeitet.